# FASCICULE DE BREVET EUROPEEN

(11) **EP 2 785 879 B1**
(45) Date de publication et mention de la délivrance du brevet: **27.07.2016**
(21) Numéro de dépôt: 12795802.3
(22) Date de dépôt: 03.12.2012
(51) Int. Cl.: C12R 1/90, A01N 63/00, A01N 63/02, C02F 3/32, C02F 103/02

(54) **PROCÉDÉ DE LUTTE BIOLOGIQUE CONTRE LES LISTERIA**
VERFAHREN ZUR BIOLOGISCHEN KONTROLLE VON LISTERIEN
METHOD FOR THE BIOLOGICAL CONTROL OF LISTERIA

(30) Priorité: 02.12.2011 FR 1161111
(43) Date de publication de la demande: 08.10.2014
(73) Titulaire: Amoeba, 69680 Chassieu (FR)
(72) Inventeur: PLASSON, Fabrice, F-69003 Lyon (FR); BODENNEC, Séléna, F-38630 Les Avenières (FR)
(74) Mandataire: Cabinet Plasseraud
(86) Numéro de dépôt international: PCT/EP2012/074248
(87) Numéro de publication internationale: WO 2013/079722

(56) Documents cités:
- WO-A2-2008/043969
- ALISHA AKYA ET AL: "Viability of Listeria monocytogenes in co-culture with Acanthamoeba spp", FEMS MICROBIOLOGY ECOLOGY, vol. 70, no. 1, 1 octobre 2009 (2009-10-01), pages 20-29, XP055030177, ISSN: 0168-6496, DOI: 10.1111/j.1574-6941.2009.00736.x cité dans la demande
- RAFIK DEY ET AL: "Free-living freshwater amoebae differ in their susceptibility to the pathogenic bacterium Legionella pneumophila", FEMS MICROBIOLOGY LETTERS, vol. 290, no. 1, 1 janvier 2009 (2009-01-01), pages 10-17, XP055030176, ISSN: 0378-1097, DOI: 10.1111/j.1574-6968.2008.01387.x

## Description

La présente invention concerne un nouveau procédé de lutte biologique contre les *Listeria* et en particulier *Listeria monocytogenes,* ainsi qu'une nouvelle composition destinée à lutter contre la prolifération de *Listeria monocytogenes.*

*Listeria monocytogenes* est une bactérie Gram positif appartenant à la famille des Listeriaceae. Cette bactérie est responsable chez l'homme des listérioses au pronostique souvent fatal (10). Chez les survivants de graves séquelles sont souvent observées. La bactérie pathogène peut traverser la barrière intestinale et la barrière placentaire, pouvant alors provoquer des infections du foetus, du nouveau-né, ou des accouchements prématurés. *Listeria monocytogenes* est aussi une cause majeure d'infection neuroinvasive avec une prévalence en augmentation au cours de ces dernières années (5) (10). Aussi, la surveillance et la prévention des listérioses constituent une préoccupation de plus en plus importante.

*Listeria monocytogenes* est une bactérie à la répartition/distribution ubiquitaire: elle est présente dans le sol, l'eau, en épiphyte sur les végétaux, etc. Très résistantes, aux traitements de nettoyage-désinfection, elle peut ainsi persister dans les ateliers de production de l'industrie agro-alimentaire par exemple ou réseaux de distribution d'eaux ou de climatisation par exemple.

De façon générale, et malgré l'importance médicale de cette bactérie, les connaissances sur l'écologie des *Listeria* demeure relativement limitée (1). Toutefois il est connu que dans l'environnement, *Listeria monocytogenes* présente une répartition ubiquitaire (15), puisque cette bactérie a été isolée du sol, d'eaux d'égouts ou d'eaux de rejets industriels (4), caractéristique qu'elle partage avec les amibes libres. De plus, la capacité de résistance de *Listeria monocytogenes* à la destruction par les macrophages humains a permis à certains scientifiques d'émettre l'hypothèse que cette bactérie pathogène pourrait résister aux amibes libres dans l'environnement, par analogie des connaissances acquises sur les relations bactéries parasites et hôtes amibiens (6). Ainsi Ly et Müller ont suggéré que *Listeria* pouvaient être résistantes aux amibes libres (6, 9). Ces hypothèses ont pu être vérifiées lorsque ces auteurs ont démontré que *Listeria monocytogenes* était capable de proliférer en présence d'amibes libres appartenant au genre *Acanthamoeba* (9) (6). De plus un effet cytotoxique de *Listeria monocytogenes* sur ses hôtes amibiens a été démontré puisqu'un enkystement *d'Acanthamoeba* est observé lorsque ces dernières sont placées en coculture avec la bactérie pathogène (6, 9). Zhou et al ont aussi pu montrer que *Listeria monocytogenes* présentait la faculté de résister aux amibes libres *Acanthamoeba castellanii:* (16). Il a aussi été démontré que *Listeria monocytogenes* est capable de croître en présence de matériel biologique relargué par les amibes lors de leur enkystation ou de leur lyse (1). Les auteurs de cette observation avaient suggéré que ces facteurs pouvaient présenter un terrain favorable aux *Listeria* pour leur maintient et/ou prolifération dans l'environnement (1). D'autres observations réalisées par Pushkareva et Ermolaeva ont permis de démontrer chez un autre protozoaire libre (*Tetrahymena pyriformis*) que *Listeria monocytogenes* était efficacement internalisé (11). L'infestation de *Tetrahymena pyriformis* par *Listeria monocytogenes* induit l'enkystement du protozoaire (11). Les données obtenues par ces chercheurs ont aussi montré que les *Listeria monocytogenes* internalisées dans ces kystes de protozoaires demeurent viables, conservent leur virulence et sont capables de causer des infections dans des modèles animaux (11).

Il apparaît donc clairement que les protozoaires et amibes libres constituent un élément important de l'écologie de *Listeria monocytogenes,* en favorisant son maintien, sa croissance dans l'environnement et en favorisant l'émergence de traits de virulence bactérienne. De plus, la capacité de Listeria à infecter les protozoaires et de survivre dans leurs formes kystiques (11) est un puissant indicateur que les protozoaires sont des facteurs favorisant la résistance des *Listeria* aux traitements biocides actuellement utilisés. Les Kystes amibiens présentent en effet une très grande résistance aux traitements biocides chimiques et/ou physiques actuellement employés (7, 8, 14). Les traitements biocides actuellement employés pour prévenir le risque *Listeria* ne donnent pas satisfaction car, au delà du développement de résistances intrinsèques à la bactérie *per se* (12, 13), les bactéries présentes à l'intérieur des protozoaires et dans les biofilms (2) sont relativement protégées, et vont donc continuer à proliférer/coloniser leur milieu.

Dans ce contexte, les inventeurs ont mis en évidence, de façon totalement inattendue, que le genre amibien *Willaertia magna* éradique les bactéries *Listeria monocytogenes.* Cet effet biocide, est doublé de la capacité de prédation déjà démontré de *Willaertia magna* envers d'autres agents amibiens qui peuvent servir de vecteur à *Listeria monocytogenes* (3)*.*

La présente invention a donc tout d'abord pour objet un procédé de lutte contre la prolifération des *Listeria* et en particulier *Listeria monocytogenes,* qui utilise des protozoaires du genre *Willaertia magna.* Les procédés conformes à l'invention n'incluent pas les méthodes de traitement appliquées au corps humain ou animal. Dans le procédé selon l'invention, le plus souvent c'est un flux gazeux ou liquide qui est traité avec des protozoaires du genre *Willaertia magna,* et en particulier de l'espèce *Willaertia magna.*

Au sens de l'invention, par *Listeria* on entend toute espèce de *Listeria* et en particulier *Listeria monocytogenes.*

Le procédé selon l'invention trouve, en particulier son application, dans la désinfection des réseaux de distribution d'eaux sanitaires ou d'eaux industrielles, des circuits de refroidissements des installations industrielles, ou des réseaux de climatisation, ou comme désinfectant de surface. Les protozoaires pourront être directement ajoutés aux eaux ou liquides circulant dans les canalisations ou réseaux à traiter. Il est également possible de les pulvériser, par exemple sous la forme d'une solution aqueuse en aérosol, dans les réseaux, cheminées, installations et surfaces industrielles à désinfecter.

De façon avantageuse, les protozoaires utilisés dans le cadre de l'invention correspondent, à la souche déposée le 26 août 2006 sous le numéro **PTA 7824** à l'ATCC, ou à la souche déposée le 26 août 2006 sous le numéro **PTA 7825** à l'ATCC, ces deux souches ayant été déposées aux noms de CENTRE NATIONAL DE LA RECHERCHE SCIENTIFIQUE (CNRS) - 3 rue Michel Ange - 75794 PARIS CEDEX 16 / FRANCE - et UNIVERSITE LYON 1 CLAUDE BERNARD - 43 Boulevard du 11 Novembre 1918 - 69622 VILLEURBANNE Cedex / FRANCE.

Les protozoaires appartenant au genre *Willaertia* correspondant à la souche déposée sous le numéro **PTA 7824** à l'ATCC ou à la souche déposée sous le numéro **PTA 7825** à l'ATCC font partie intégrante de l'invention. Lesdites souches déposées **PTA 7824** et **PTA 7825** sont également décrites dans la publication de la demande internationale PCT WO2008/043969.

De tels protozoaires pourront donc être utilisés dans des agents désinfectants, en particulier destinés à l'élimination des bactéries *Listeria* et en particulier *Listeria monocytogenes* et à lutter contre la prolifération et la contamination par listériose.

De façon avantageuse, l'agent désinfectant selon l'invention se présente sous la forme d'une solution ou d'une suspension aqueuse, par exemple dans de l'eau distillée. L'agent désinfectant pourra se présenter sous une forme pulvérisable, par exemple en aérosol ou tout autre moyen d'application.

L'activité des protozoaires du genre *Willaertia,* et en particulier de l'espèce *Willaertia magna,* inhibant la prolifération de *Listeria monocytogenes* a été mise en évidence par les inventeurs en comparant la réplication de *Listeria monocytogenes* chez les genres *Acanthamoeba* et *Hartmannella* utilisés en tant que modèles amibiens avec celle observée chez le genre amibien *Willaertia.*

Etant donné le rôle essentiel joué par les amibes dans la prolifération et le maintien des *Listeria* et notamment des *Listeria monocytogenes* dans le milieu extérieur, éléments qui conditionnent l'épidémiologie de la listériose puisqu'il n'existe pas de transmission interhumaine, le procédé et l'agent désinfectant selon l'invention présentent de nombreux avantages, en termes de coût, d'efficacité et de respect de l'environnement, notamment.

Les exemples ci-après permettent d'illustrer l'invention mais n'ont aucun caractère limitatif.

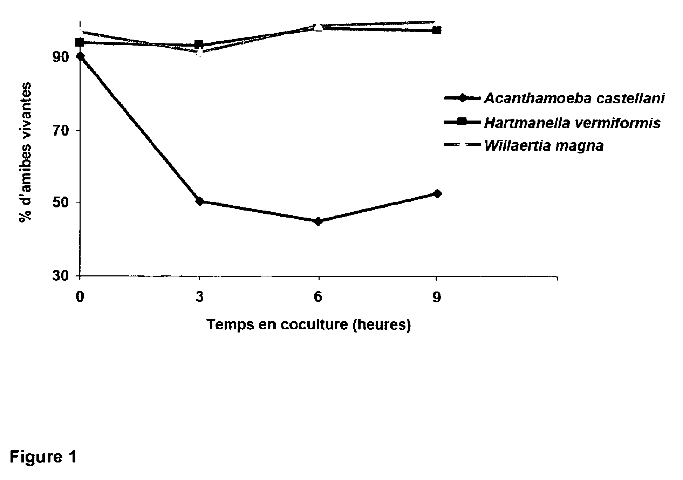

La **Figure 1** montre l'évolution spontanée des populations respectives d'amibes *Hartmannella, Acanthamoeba* et *Willaertia* après mise en coculture avec *Listeria monocytogenes* à un ratio amibe/bactérie initial de 10.

Les différentes amibes libres sont mises en cocultures (temps 0 heure) avec *Listeria monocytogenes* à un ratio de 10 (10 bactéries / 1 amibe) comme décrit dans la partie matériels et méthodes. Des aliquots des suspensions cellulaires sont ensuite prélevés toutes les 3 heures suivant la mise en coculture et le pourcentage d'amibes vivante est déterminé par un test d'exclusion au bleu trypan et observation microscopique à l'aide d'une cellule de Mallasez. Les données sont exprimées en % de cellules vivantes, négatives au test d'exclusion au bleu trypan.

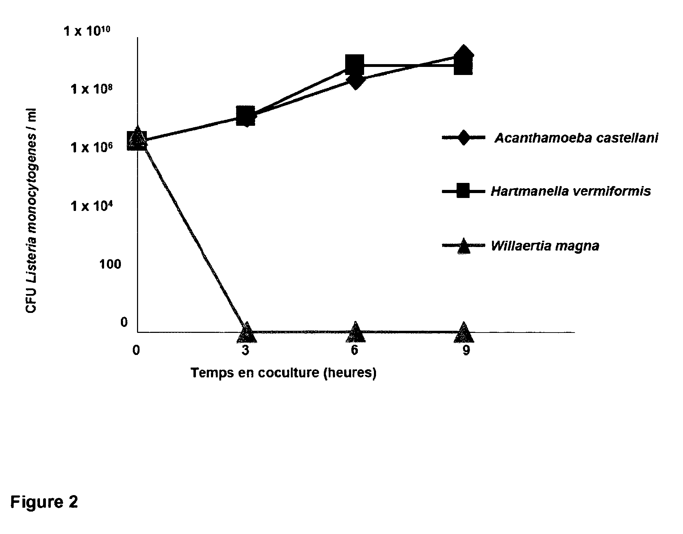

La **Figure 2** montre les cinétiques comparées du développement de *Listeria monocytogenes* obtenu en coculture avec différents genres amibiens, dont le genre *Willaertia.*

Les différentes amibes libres sont séparément mises en cocultures (temps 0 heure) avec *Listeria monocytogenes* à un ratio de 10 (10 bactéries / 1 amibe). Des aliquots des suspensions cellulaires sont ensuite prélevés toutes les 3 heures suivant la mise en coculture et les concentrations en *Listeria monocytogenes* déterminées comme décrit dans la partie matériels et méthodes.

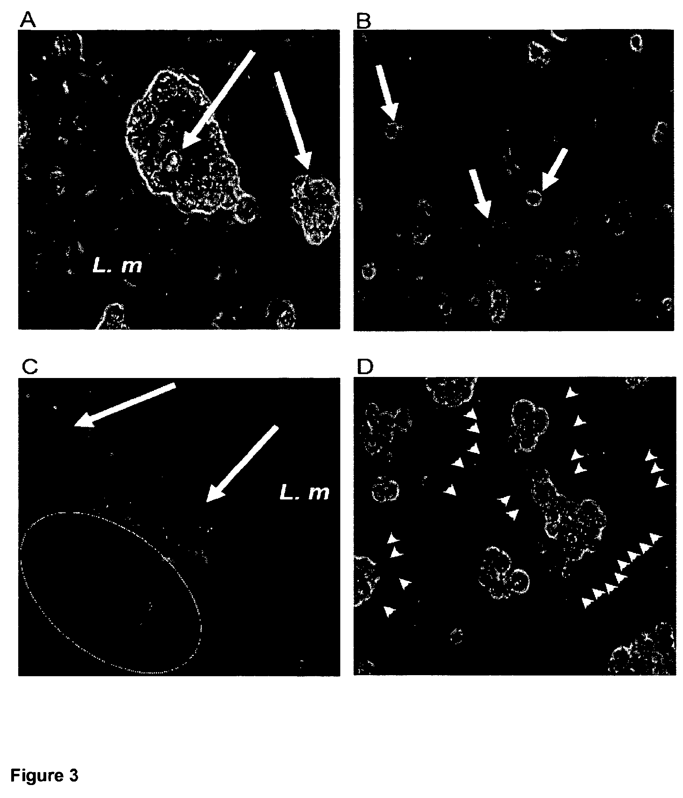

**La** **Figure 3** montre l'effet biocide des *Willaertia magna* sur un biofilm formé par *Listeria monocytogenes.* Cette figure montre aussi l'absence d'effet des amibes libres *Acantlvameoba castellanii* et *Hartmanella vermiformis.*

Les *Listeria monocytogenes* ont été ensemencées sur gélose TSA, qui sont incubées à 30°C pendant 48 heures afin d'assurer la formation d'un biofilm dense de bactérie sur la gélose. Puis 1 × 10⁵ amibes (*Acanthamoeba castellanii,* **panel A ;** *Hartmarzella vermiformis,* **panel B ;** *Willaertia magna,* **panels C et D)** resuspendues dans 100 µl d'eau désionisée stérile sont ensemencées au centre de la gélose. Après 24 heures à 30°C les géloses sont examinées sous microscope afin de déterminer l'aspect des amibes et l'influence de celles-ci sur le biofilm. **A :** les flêches blanches montrent l'aspect des groupes de kystes formés par *Acanthamoeba castellanii* en présence de *Listeria monocytogenes* **(L. m.).** Noter l'absence de plage de lyse du tapis bactérien autour des groupes de kystes amibiens. **B :** Noter la présence des nombreuses formes kystiques d'*Hartmanella vermiformis,* dont quelques exemples sont indiqués par les flêches blanches. Noter aussi la très forte densité de *Listeria monocytogenes* entourant les kystes d'*Hartmarzella vermiformis* et l'absence totale de plages de lyse du biofilm bactérien. C : Effet des *Willaertia magna* sur le biofilm formé par *Listeria monocytogenes.* Les flêches blanches indiquent des groupes de trophozoïtes de *Willaertia magna* situés au front d'une plage de lyse du biofilm. Noter l'absence de film bactérien (zone délimitée par le cercle blanc dans le coin gauche inférieur de la photomicrographie) en amont des groupes de *Willaertia magna* et le film dense bactérien (indiqué par les initiales **L. m.)** en aval des groupes d'amibes (coin droit supérieur de la photomicrographie). **D :** autre photomicrographie montrant la formation des plages de lyse du biofilm formé par *Listeria monocytogenes.* Noter les groupes de *Willaertia magna* et les front de plages de lyse (indiqués par les pointes blanhes) du biofilm bactérien.

### 1. MATERIEL ET METHODES

### 1.1. Souches utilisées:

***Listeria monocytogenes :*** la souche utilisée est la souche CL 3970 (Oxoid, France).
- . Elle est entretenue sur gélose TSA (Tryptone Soja Agar) (ref PO 5012, Oxoid, France) à raison d'un repiquage par semaine. La souche est ensemencée en stries larges sur boîte de gélose TSA et incubée 2 jours à 30°C.
- ***Amibes :*** les souches utilisées appartiennent à trois espèces amibiennes différentes :
   ∘ ***Hartmannella vermiformis,***
   ∘ *Acanthamoeba castellanii,* souches déposée à ATCC sous les n°30010
   ∘ *Willaertia magna* (souches déposées à ATCC sous les n° PTA7824 et PTA 7825),

Ces trois souches sont cultivées axéniquement , en présence de 10% de sérum de veau foetal, sur milieu SCGYEM (Serum Casein Glucose Yeast Extract Medium), réparti en tubes FALCON^{®} (3033) à raison de 3ml par tube. En entretien, les formes végétatives sont repiquées tous les 8-9 jours. Pour les cocultures, on utilise des repiquages de 3 à 4 jours de manière à disposer de trophozoites en pleine phase de croissance exponentielle.
Le milieu SCGYEM est obtenu comme suit :

| | |
|---|---|
| ➢ Caséine (MERCK 1.02244.010) | 10 g |
| ➢ Na₂HPO₄ | 1,325 g |
| ➢ KH₂PO₄ | 0,8 g |
| ➢ Glucose | 2,5 g |
| ➢ Extrait de levure (DIFCO 0127-17-9) | 5 g |
| ➢ Eau distillée | 900 ml |
| ➢ Sérum de veau foetal | 100 ml |

Aux 900 ml d'eau distillée, on ajoute 2,5 ml de NaOH (1N), puis Na₂HPO₄ et KH₂PO₄ On chauffe légèrement sur plaque chauffante, puis on ajoute progressivement la caséine sous agitation magnétique. Après dissolution de la caséine, on incorpore le glucose et l'extrait de levure.

Après dissolution complète, on filtre successivement sur fibre de verre (SARTORIUS SM 6513400), puis sur membrane de 1µm (WHATMAN 7190 004). Le milieu est ensuite aliquoté en flacons de verre. Les flacons sont stérilisés à l'autoclave 20 minutes à 120°C. Avant l'utilisation définitive et répartition du milieu, on ajoute stérilement sous hotte à flux laminaire le sérum de veau foetal à raison de 10% du volume final.

### 1.2. Coculture monoamibienne de Listeria monocytogenes.

### 1.2.1. Préparation de l'inoculum bactérien:

A partir d'une culture de 2 jours sur gélose TSA, on prépare une suspension de *Listeria monocytogenes* dans de l'eau distillée stérile de façon à obtenir 1 unité de Densité Optique à 550nm, soit une concentration de 10⁹ UFC (Unité formant colonie) /ml.

### 1.2.2. Réalisation des cocultures mono-amibiennes

Les cocultures sont réalisées dans des tubes pour cultures cellulaires (FALCON^{®} 3033) contenant 3 ml d'eau stérilisée par autoclave. L'ensemencement des tubes se fait à raison de 1 × 10⁵ amibes/ml, à partir d'une suspension amibienne axénique préalablement dénombrée sur un hématimètre de Malassez. L'infestation des amibes par *Listeria monocytogenes* est réalisée en fixant un ratio *Listeria monocytogenes* / amibe de 10, soit 1 × 10⁶ bactéries / ml de milieu d'incubation. Aussitôt après l'infestation, les tubes de cocultures sont centrifugés à faible vitesse (760g pendant 10 min) pour favoriser le contact entre amibes et bactéries. Après 10 min, les tubes sont remis en suspension manuellement et sont incubés, en position inclinée, à l'étuve à 30°C.

Les devenirs des amibes et de *Listeria monocytogenes* mis en coculture sont déterminés de la façon suivante :
Les cocultures sont suivies pendant 9 heures après l'infestation bactérienne. A chaque intervalle de temps (toutes les 3 heures), les tubes de coculture sont prélevés et examinés à la fois du point de vue amibien et bactérien après agitation vigoureuse au vortex afin de détacher les amibes des parois. Pour chaque tube examiné:
   - La numération des amibes s'effectue directement sur cellule de Malassez.
   - La détermination des concentrations en *Listeria monocytogenes* est effectuée par étalement direct du milieu de culture sur gélose TSA après dilution de 10 en 10 en eau distillée stérile, dans des microtubes Eppendorf. Chaque dilution est étalée en triplicat sur gélose TSA à raison de 100µl par boîte. Les boîtes sont alors mises à incuber à 30°C pendant 48 heures au minimum. Une première lecture des géloses TSA s'effectue 24 heures après l'étalement en dénombrant les colonies; elle est suivie d'une deuxième lecture au 2^{ème} jour pour confirmation. Les concentrations de *Listeria monocytogenes* sont exprimées en UFC / ml de milieu d'incubation en tenant compte du facteur de dilution et en supposant que chaque colonie correspond à 1 bactérie initialement présente dans la suspension diluée.

Pour chaque genre amibien, les courbes de croissance de *Listeria monocytogenes,* sont représentées en fonction du temps.

De plus, l'éventuel effet cytotoxique de *Listeria monocytogenes* sur les différentes espèces d'amibes est déterminé de la façon suivante :
- en comptant la proportion d'amibes présentant une positivité au test d'exclusion au bleu trypan. Ce test est réalisé sous microscope en comptant sous cellule de Malassez le nombre de cellules positives au bleu trypan / au nombre de cellules totales.
- En déterminant la propension des amibes à s'enkyster en présence de *Listeria monocytogenes.*

### 1.3. Effet de Willaertia magna sur les biofilm de Listeria monocytogenes.

Les biofilms de *Listeria monocytogenes* sont formés de la façon suivante : une quantité déterminée de *Listeria monocytogenes* dans 100 µl d'eau stérile est déposée et étalée sur une gélose TSA. Les géloses sont placées à 30°C pendant 48 heures de façon à permettre le développement d'un film bactérien dense et uniforme sur l'ensemble de la surface de la gélose. Puis 1 × 10⁵ amibes ( *Acanthamoeba castellanii*, *Hartmarzella vermiformis* ou *Willaertia magna)* sont déposées au centre de la gélose qui est placée pendant 24 heures à 30°C. Les géloses sont ensuite observées au microscope optique (grossissement × 400) afin d'y détecter la formation d'éventuelles plages de lyse du tapis bactérien.

### 2. RESULTATS

### 2.1. Willaertia magna présente une résistance à Listeria monocytogenes.

L'effet de *Listeria monocytogenes* sur la survie des différentes espèces amibiennes testées a été déterminé par un test d'exclusion au bleu trypan. Très rapidement après mise en coculture d'*Acanthamoeba castellanii* avec la bactérie, il apparaît un effet cytotoxique majeur chez cette espèce amibienne, avec une chute de ∼ 50% de la viabilité après 3heures de coculture (voir **Figure 1****).** A l'opposé ce phénomène n'est jamais observé lorsque *Willaertia magna* est mis en coculture avec *Listeria monocytogenes,* y compris j'usqu'à 9 heures d'incubation avec une viabilité se maintenant proche des 100% **(****Figure 1****).** Comme *Willaertia magna,* l'amibe libre *Hartmanella vermiformis* ne présente pas de chute en terme de viabilité déterminée par exclusion au bleu trypan **(****Figure 1****).** Toutefois, l'examen microscopique des cocultures amibiennes-*Listeria monocytogenes* démontre une forte propension à un enkystement chez *Hartmanella vermiformis* et chez les formes viables d'*Acanthamoeba castellanii* (voir **Table 1).** Ce phénomène d'enkystement n'est jamais observé chez *Willaertia magna* lorsque placé en coculture avec la bactérie pathogène **(Table 1).**

**Table 1. Effet de Listeria monocytogenes sur l'induction de formes kystiques chez les différentes espèces d'amibes libres.**

| | **Temps en coculture (heures)** | | | |
|---|---|---|---|---|
| | **0** | **3** | **6** | **9** |
| ***Harmanella vermiformis*** | **ND** | **+** | **++** | **++** |
| ***Acanthamoeba castellanii*** | **ND** | **ND** | **+** | **+++** |
| ***Willaertia magna*** | **ND** | **ND** | **ND** | **ND** |

| | | | | |
|---|---|---|---|---|
| Les amibes libres sont mises en cocultures (temps 0 heure) avec *Listeria monocytogenes* à un ratio de 10 (10 bactéries / 1 amibe) comme décrit dans la partie matériels et méthodes. Des aliquots des suspensions cellulaires sont ensuite prélevés toutes les 3 heures suivant la mise en coculture comme indiqué sur le tableau ci-dessus. La densité de kystes amibiens est exprimée de la façon suivante : **ND :** kistes non détectés ; + : présence de kystes (proportion inférieure à 10% des formes viables) ; ++ : présence de kystes (proportion comprise entre 10% et 30% des formes viables) ; +++ : présence de kystes (proportion supérieure à 30% des formes viables). | | | | |

L'ensemble de ces observations (pas d'enkystement et pas de cytotoxicité induite par *Listeria monocytogenes*) démontrent clairement que *Willaertia magna,* au contraire des autres espèces amibiennes, présente la faculté originale de résister à *Listeria monocytogenes.*

### 2.2. Prédation de Listeria monocytogenes par Willaertia magna

Les résultats des cocultures de *Listeria monocytogenes* réalisées en présence d'amibes appartenant aux genres *Hartmannella* et *Acanthamoeba* démontrent une importante multiplication de la bactérie en présence de ces deux genres amibiens puisqu'on note une augmentation atteignant ∼ 3 log des concentrations bactériennes en 9 heures (voir **Figure 2****).** A l'inverse, alors que les cocultures sont réalisées dans des conditions strictement identiques, on constate en présence de l'amibe *Willaertia magna* une disparition totale des *Listeria monocytogenes* détectables (voir **Figure 2****).** La chute mesurée des concentrations en *Listeria monocytogenes* est de ∼ 6 Log en 3 heures, démontrant un effet massif de prédation des *Willaertia magna* envers *Listeria monocytogenes.*

Cet effet des *Willaertia magna* sur les *Listeria monocytogenes* est encore illustré **Figure 3****.** Ainsi, après 24 heures en présence des *Willaertia magna,* apparaissent très clairement des surfaces de la gélose où le tapis bactérien a disparu (ces zones sont appelées ici plages de lyse du tapis/biofilm bactérien). L'examen microscopique montre aussi que les *Willaertia magna* sont concentrées à la limite de cette plage de lyse ; cet effet est illustré **Figure 3** **panel C.** La destruction du tapis bactérien par *Willaertia magna* est aussi illustrée sur le **panel D de la** **Figure 3** où on distingue clairement des groupes de l'amibe entourés d'un tapis bactérien détruit ou en cour de destruction. A l'inverse, en présence *d'Acanthamoeba castellanii* ou d'*Hartmarzella vermiformis,* ce phénomène n'a jamais pu être observé. L'examen microscopique des géloses montre que les amibes *Acanthamoeba castellanii* et *Hartmanella vermiformis* s'enkystent rapidement lorsque déposées sur le film de *Listeria monocytogenes.* Ce phénomène est illustré panels **A et B** de la **Figure 3****.** On note aussi l'absence totale de plage de lyse du tapis bactérien autour des kystes *d'Acanthamoeba castellanii* et d'*Hartmanella vermiformis,* au contraire du phénomène observé avec *Willaertia magna.* L'ensemble de ces données et observations montrent clairement l'effet de prédation de *Willaertia magna* envers la bactérie pathogène *Listeria monocytogenes.*

### Références bibliographiques

1. Akya A, Pointon A, and Thomas C. Viability of Listeria monocytogenes in co-culture with Acanthamoeba spp. FEMS Microbiol Ecol 70: 20-29, 2009.
2. Belessi CE, Gounadaki AS, Psomas AN, and Skandamis PN. Efficiency of different sanitation methods on Listeria monocytogenes biofilms formed under various environmental conditions. Int J Food Microbiol 145 Suppl 1: S46-52, 2011.
3. Bodennec J, Dey R, and Pernin P. Novel method for biologically combating the prolifération of Legionella pneumophila, and novel disinfecting agent containing amoebic protozoa of the Willaertia genus. edited by University CBL. France: 2010.
4. Dijkstra RG. The occurence of Listeria monocytogenes in surface water of canals and lakes, in ditches of one big polder and in the effluents and canals of a sewage treatment plant. Zentralbl Bakteriol Mikrobiol Hyg[B] 176: 202-205, 1982.
5. Goulet V, Hedberg C, Le Monnier A, and de Valk H. Increasing incidence of listeriosis in France and other European countries. Emerg Infect Dis 14: 734-740, 2008.
6. Greub G, and Raoult D. Microorganisms résistant to free-living amoebae. Clin Microbiol Rev 17: 413-433, 2004.
7. Khunkitti W, Lloyd D, Furr JR, and Russell AD. Acanthamoeba castellanii: growth, encystment, excystment and biocide susceptibility. J Infect 36: 43-48, 1998.
8. Lloyd D, Turner NA, Khunkitti W, Hann AC, Furr JR, and Russell AD. Encystation in Acanthamoeba castellanii: development of biocide résistance, J Eukaryot Microbiol 48: 11-16, 2001.
9. Ly TMC, and Müller HE. Ingested Listeria monocytogenes survive and multiply in protozoa. J Med Microbiol 33: 51-54, 1990.
10. Mailles A, Lecuit M, Goulet V, Leclercq A, and Stahl JP. Listeria monocytogenes encephalitis in France. Med Mal Infect In Press.
11. Pushkareva VI, and Ermolaeva SA. Listeria monocytogenes virulence factor Listeriolysin O favors bacterial growth in co-culture with the ciliate Tetrahymena pyriformis, causes protozoan encystment and promotes bacterial survival inside cysts. BMC Microbiol 10: 26, 2010.
12. Rajkovic A, Smigic N, Uyttendaele M, Medic H, de Zutter L, and Devlieghere F. Résistance of Listeria monocytogenes, Escherichia coli 0157:H7 and Campylobacter jejuni after exposure to repetitive cycles of mild bactericidal treatments. Food Microbiol 26: 889-895, 2009.
13. Rakic-Martinez M, Drevets DA, Dutta V, Katic V, and Kathariou S. Listeria monocytogenes strains selected on ciprofloxacin or the disinfectant benzalkonium chloride exhibit reduced susceptibility to ciprofloxacin, gentamicin, benzalkonium chloride and other toxic compounds. Appl Environ Microbiol In Press, 2011.
14. Thomas V, Bouchez T, Nicolas V, Robert S, Loret JF, and Levi Y. Amoebae in domestic water systems: résistance to disinfection treatments and implication in Legionella persistence. JAppl Microbiol 97: 950-963, 2004.
15. Weis J, and Seeliger HP. Incidence of Listeria monocytogenes in nature. Appl Microbiol 30: 29-32, 1975.
16. Zhou X, Elmose J, and Call DR. Interactions between the environmental pathogen Listeria monocytogenes and a free-living protozoan (Acanthamoeba castellanii). Environ Microbiol 9: 913-922, 2007.

## Revendications

1. Procédé de lutte contre la prolifération de *Listeria monocytogenes,* à l'exception des méthodes de traitement appliquées au corps humain ou animal, **caractérisé en ce qu'**il utilise des protozoaires de l'espèce Willaertia magna.

2. Procédé selon la revendication 1 **caractérisé en ce que** l'on traite un flux gazeux ou liquide, ou une surface solide avec des protozoaires de l'espèce *Willaertia magna.*

3. Procédé selon la revendication 1 ou 2 **caractérisé en ce que** ces protozoaires correspondent, à la souche déposée sous le numéro **PTA 7824** à l'ATCC ou à la souche déposée sous le numéro **PTA 7825** à l'ATCC.

4. Procédé selon l'une des revendications 1 à 3 **caractérisé en ce qu'**il est mis en oeuvre pour la désinfection des réseaux de distribution d'eaux sanitaires ou d'eaux industrielles, des circuits de refroidissements des installations industrielles, ou des réseaux de climatisations, ou de toutes surfaces industrielles.

5. Procédé selon l'une des revendications 1 à 3 **caractérisé en ce qu'**il est mis en oeuvre pour lutter contre la formation de biofilms dans les canalisations d'eaux, les surfaces en contact ou non avec denrées alimentaires humaine ou animale.

6. Utilisation d'un agent désinfectant contenant des protozoaires de l'espèce Willaertia magna comme biocide sur les *Listeria* à l'exception d'une utilisation pour le traitement appliqué au corps humain ou animal.

7. Utilisation selon la revendication 6, **caractérisée en ce que** les protozoaires correspondent à la souche déposée sous le numéro **PTA 7824** à l'ATCC ou à la souche déposée sous le numéro **PTA 7825** à l'ATCC.

8. Utilisation selon la revendication 6 ou 7, **caractérisé en ce qu'**il se présente sous la forme d'une solution ou d'une suspension aqueuse.

## Patentansprüche

1. Verfahren zur Bekämpfung der Proliferation von *Listeria monocytogenes* mit Ausnahme von Behandlungsverfahren, die auf den menschlichen oder tierischen Körper angewendet werden, **dadurch gekennzeichnet, dass** es Protozoen der Spezies Willaertia magna verwendet.

2. Verfahren nach Anspruch 1, **dadurch gekennzeichnet, dass** man einen Gas- oder Flüssigkeitsstrom oder eine feste Oberfläche mit Protozoen der Spezies *Willaertia magna* behandelt.

3. Verfahren nach Anspruch 1 oder 2, **dadurch gekennzeichnet, dass** diese Protozoen dem unter der Nummer **PTA 7824** bei der ATCC hinterlegten Stamm oder dem unter der Nummer **PTA 7825** bei der ATCC hinterlegten Stamm entsprechen.

4. Verfahren nach einem der Ansprüche 1 bis 3, **dadurch gekennzeichnet, dass** es zur Desinfektion von Hausabwasser- oder Industrieabwasserverteilungsnetzen, Kühlkreisläufen von Industrieanlagen oder Kühlnetzen oder allen industriellen Oberflächen verwendet wird.

5. Verfahren nach einem der Ansprüche 1 bis 3, **dadurch gekennzeichnet, dass** es zur Bekämpfung der Bildung von Biofilmen in Abwasserkanälen, Oberflächen, die mit menschlichen oder tierischen Lebensmitteln in Kontakt sind oder nicht, verwendet wird.

6. Verwendung eines Protozoen der Spezies Willaertia magna enthaltenden Desinfektionsmittels als Biozid gegen *Listeria* mit Ausnahme einer Verwendung für eine auf den menschlichen oder tierischen Körper angewendete Behandlung.

7. Verwendung nach Anspruch 6, **dadurch gekennzeichnet, dass** die Protozoen dem unter der Nummer **PTA 7824** bei der ATCC hinterlegten Stamm oder dem unter der Nummer **PTA 7825** bei der ATCC hinterlegten Stamm entsprechen.

8. Verwendung nach Anspruch 6 oder 7, **dadurch gekennzeichnet, dass** sie in Form einer Lösung oder einer wässrigen Suspension vorliegt.

## Claims

1. A method for controlling the proliferation of *Listeria monocytogenes*, with the exception of the treatment methods applied to the human or animal body, **characterized in that** it uses protozoa of the *Willaertia magma* species.

2. The method as claimed in claim 1, **characterized in that** a gas or liquid stream or a solid surface is treated with protozoa of the *Willaertia magna* species.

3. The method as claimed in claim 1 or 2, **characterized in that** these protozoa correspond to the strain deposited under number **PTA 7824** at the ATCC or to the strain deposited under number **PTA 7825** at the ATCC.

4. The method as claimed in one of claims 1 to 3, **characterized in that** it is implemented for the disinfection of sanitation water or industrial water distribution networks, cooling circuits for industrial plants, or air-conditioning networks, or any industrial surfaces.

5. The method as claimed in one of claims 1 to 3, **characterized in that** it is implemented for controlling the formation of biofilms in water pipes, or surfaces possibly in contact with human or animal food products.

6. The use of a disinfecting agent containing protozoa of the *Willaertia magna* species, as a biocide on *Listeria,* with the exception of a use for the treatment applied to the human or animal body.

7. The use as claimed in claim 6, **characterized in that** the protozoa correspond to the strain deposited under number **PTA 7824** at the ATCC or to the strain deposited under number **PTA 7825** at the ATCC.

8. The use as claimed in claim 6 or 7, **characterized in that** it is in the form of an aqueous solution or suspension.
